# EUROPEAN PATENT SPECIFICATION

(11) **EP 1 668 147 B1**
(45) Date of publication and mention of the grant of the patent: **02.01.2008**
(21) Application number: 04785216.5
(22) Date of filing: 23.09.2004
(51) Int. Cl.: C12Q 1/66, C12Q 1/00, C12G 1/04

(54) **DETECTION OF LIVING CELLS IN POLYMERS OR PIGMENTS**
NACHWEIS LEBENDER ZELLEN IN POLYMEREN ODER PIGMENTEN
DETECTION DE CELLULES VIVANTES DANS DES POLYMERES OU DES PIGMENTS

(30) Priority: 29.09.2003 US 673895
(43) Date of publication of application: 14.06.2006
(73) Proprietor: EASTMAN CHEMICAL COMPANY, Kingsport, TN 37660 (US)
(72) Inventor: HOCHSTETLER, Spencer, Erich, Kingsport, TN 37664 (US); MATOSKY, Andrew, Joseph, Kingsport, TN 37664 (US)
(74) Representative: Wibbelmann, Jobst
(86) International application number: PCT/US2004/031859
(87) International publication number: WO 2005/033329

(56) References cited:
- EP-A- 0 816 512
- EP-A- 1 134 291
- WO-A-00/46392
- US-A- 4 421 848
- US-A- 5 055 397

## Description

### FIELD OF THE INVENTION

This invention pertains to a process for detection of living cells in aqueous mixtures of polymers or pigments. More specifically, this invention pertains to a process for releasing adenosine triphosphate from living cells in aqueous mixtures of polymers or pigments and a process for the detection of the ATP thus released. The invention further pertains to a kit for the detection of living cells in aqueous mixtures of polymers.

### BACKGROUND OF THE INVENTION

Aqueous mixtures of polymers or pigments, in particular, aqueous dispersions of polymers or pigments which are used in the manufacture of paints, coatings, cosmetics, adhesives, polishes, etc., are subject to contamination by microorganisms. Such contamination presents a number of serious difficulties to the coatings and cosmetics industries. For example, aqueous mixtures of polymers contaminated with microorganisms often exhibit unpleasant odors which may make the final product unfit for use. Contaminated dispersions may exhibit a reduction in molecular weight and altered flow properties, which can render them unfit for their intended applications. If printing paper is coated with contaminated dispersions, the paper may tear on the high speed machines used in print shops as a result of the compromised properties of the dispersion. Films produced with contaminated dispersions can become brittle or discolored. Dispersions contaminated with microorganisms often form gas bubbles which, in turn, can cause the formation of pinholes in the final coating. Organic acids are often produced by microorganisms and can corrode storage tanks, drums or other metal objects.

To address this problem, aqueous mixtures of polymers or pigments are tested for the presence of the microorganisms by way of growth tests or culturing methods such as, for example, Easycult-TTC™, available from Orion Diagnostics, Finland, or by culturing the microorganisms on agar plates. Bacteria, yeasts, and fungi are measured either by standard colony counting in a growth medium or by instrumental methods, such as a microscope, turbidometer, nephelometer, and the like. Somatic cells often are counted with particle counters, such as an electro-optical particle counter, by instruments based on detecting fluorescent particles, by indirect measurement based on the quantity of a metabolic product, or by microscopy. These methods require either complex, expensive equipment or are often inaccurate because of interference from non-cellular particles or from the various additives and chemicals present in the dispersion. Further, culture or growth methods only work indirectly after addition of a culture medium and require 24 to 48 hours of incubation at elevated temperatures (approx. 35 to 37°C). These methods, therefore, suffer from the disadvantage that a long waiting period ensues between sampling and test results. In a commercial manufacturing operation, a long waiting period provides more opportunity for cross-contamination of production lots, higher inventory costs, and mistaken shipment of contaminated products. To avoid these problems, manufacturers frequently employ the preventative use of preservatives, biocides, and disinfecting agents, which are expensive and difficult to apply effectively. Such preservatives, in higher concentrations, can negatively influence the material properties of the polymeric emulsions and dispersions. In the paint, coatings, cosmetics, inks, and adhesives industries, therefore, it is important to determine promptly the level of microbial contamination of raw materials and finished products to insure quality products and prevent contamination of raw material inventories. Therefore, rapid alternatives to the conventional methods discussed above are needed.

Firefly bioluminescent measurement of adenosine triphosphate, abbreviated herein as "ATP", (see for example, U.S. Patent No.'s 3,745,090 and 3,933,592) is a rapid and sensitive method for determining the number of living cells in a sample. In this method, the living cells are lysed or ruptured to release their ATP molecules which are then reacted with the reagent luciferin in the presence of oxygen, magnesium ions, and the luciferase enzyme to produce photons. The photons are detected by a photomultiplier, photodiode, or other solid state light-sensing device to produce an electric pulse output which may be directly correlated to the presence of microorganisms in the sample. The firefly bioluminescent assay has been described as a means to detect the presence of living cells in a variety of circumstances and media such as, for example, in milk, foodstuffs, urine, spinal fluid, water, beer, cosmetics, dispersions that contain polymers and pigments, and on the surfaces of countertops (see, for-example, U.S. Patent No.'s 3,745,090; 5,736,351; 4,303,752; 4,144,134; German Patent Application No. 196 25 137 A1; Japanese Kokai Application No. 9-121896; and Nielsen et al., J. Assoc. Off. Anal. Chem. (1989), 72, 708-711. The methods thus described, however, typically rely on chemical agents, such as surfactants, to lyse or rupture the living cells to release their ATP. Chemical lysis, however, may give variable results and the lysis agent can interfere with the bioluminescent assay. In particular, bioluminescent assays using chemical lysis can be undependable for the detection of living cells in aqueous mixtures of polymers or pigments such as, for example, aqueous dispersions of paints, cosmetics, inks, and adhesives, and may not be completely trusted to give accurate results for these materials. Accordingly, a quick, sensitive, and reliable method of releasing ATP from living cells in aqueous mixtures of polymers or pigments is needed. In addition, a reliable method is needed to detect and thus avoid microbial contamination of aqueous mixtures of polymers or pigments in such products as waterborne paints, coatings, cosmetics, and adhesives.
US-A,4,421,848 describes a method for detecting the presence of live organisms in substances through an ATP assay, said disruption may be achieved by violent agitation.
EP-A-0 816 512 discloses a process for detecting living cells in an aqueous dispersion wherein the cells are dissolved by chemical lysis in the presence of surfactants.

### SUMMARY OF THE INVENTION

We have discovered that ATP may be reliably and efficiently released from living cells present in aqueous dispersions of polymers or pigments by agitation in the presence of a particulate disruption agent. Our novel process uses a particulate disruption agent which unexpectedly releases a greater quantity of ATP over chemical lysis and other mechanical disruption methods, and enables a higher sensitivity and greater accuracy for the detection of living cells by ATP assays. Thus, the present invention provides a process for detecting living cells in an aqueous dispersion of a polymer or pigment, comprising: (i) agitating said aqueous dispersion in the presence of a particulate disruption agent sufficient to cause rupturing of and thereby release ATP from said living cells and (ii) detecting said released ATP. The aqueous dispersion of polymers of pigments or the present invention may include aqueous dispersions of polymers such as, for example, vinyl polymers, polysilanes, and acrylic latexes, which are commonly used to manufacture paints, coatings, and adhesives. The disruption agent may comprise any solid particles which are harder than the walls of the living cells and may include, but are not limited to, one or more metals, metal oxides, silicon oxide, carborundum, ceramic, glass, plastic, or sand.

Our process may be used for aqueous dispersions of polymers containing a wide range of living cells including, but not limited to, plant cells, spores, yeasts, fungi, molds, and mycobacteria. Many of these living cells are difficult to culture or are resistant to lysing by chemical agent or mechanical methods such as homogenization or sonication.

Our invention also provides a kit for detecting living cells in aqueous dispersions of polymers. The invention thus provides a kit as defined in claim 17 for detecting living cells in an aqueous dispersion of a polymer or pigment comprising a disruption container comprising a particulate disruption agent therein, and a reagent container comprising a bioluminescent reagent therein. The novel process and kit of our invention provides a simple, reliable, and sensitive procedure to detect living cells in an aqueous mixture of polymers or pigments that can be performed easily in the field and requires minimal operator handling.

### DETAILED DESCRIPTION

The present invention provides a quick and reliable method for releasing ATP from living cells in an aqueous dispersion of a polymer or pigment. The released ATP may then be assayed as means for detecting living cells in aqueous mixtures of polymers such as, for example, aqueous latexes and waterborne paints, cosmetics, adhesives, inks, and personal care products. In a general embodiment, our invention provides a process for releasing adenosine triphosphate (ATP) from living cells in an aqueous mixture of a polymer or pigment comprising agitating said aqueous dispersion in the presence of a particulate disruption agent sufficient to cause rupturing of and thereby release ATP from said living cells. Our process is effective for releasing ATP from all cell types and forms and, when used in combination with ATP assays, provides a method for detecting living cells in aqueous mixtures of polymers or pigments which shows a greater accuracy and reproducibility over traditional culture or chemical lysis methods.

Unless otherwise indicated, the ranges stated in this disclosure and the claims are intended to include the entire range specifically and not just the endpoint(s). For example, a range stated to be 0 to 10 is intended to disclose all whole numbers between 0 and 10 such as, for example 1, 2, 3, 4, etc., all fractional numbers between 0 and 10, for example 1.5, 2.3, 4.57, 6.1113, etc., and the endpoints 0 and 10. Also, a range associated with chemical substituent groups such as, for example, "C₁ to C₅ hydrocarbons", is intended to specifically include and disclose C₁ and C₃ hydrocarbone as well as C₂, C₃, and C₄ hydrocarbons.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in its respective testing measurements.

ATP is present in all living cells. By the term "living-cells", it is meant, of course, that the cells are living until they are killed by the process of the present invention. The term "living cells", as used herein, is intended to be synonymous with the terms "cells", "organism" and "microorganism" and includes both single cell and multicelluar microorganisms having cell membranes, cells with rigid cell walls, cells with non-elastic, and non-rigid cell walls. Our invention is also useful for releasing ATP from non-cellular biological material such as pollen and spirulina, intra-cellular material, such as organelles and nuclei; unbounded homogenous material; or a combination thereof. The living cells of the present invention thus comprise one or more prokaryotic microorganisms including bacteria and mycobacteria; eukaryotic cells and microorganisms such as fungi and mold; plant cells, such as yeast; and even protoplasts, spheroplasts and spores; and biological materials which are not technically "cells", such as pollen and the like. Our process is particularly useful for releasing ATP from living cells from certain bacteria, molds, spores, and yeasts, and mycobacteria, which are difficult to lyse by chemical, enzymatic, or mechanical means, such as sonication, homogenization, or by the application of high pressure as in the "French Press". The living cells of the invention may comprise one or more microorganisms such as, for example, one or more bacteria selected from the-genus *Bacillus sp., Lactobacillus sp., Citrobacter sp., Serratia sp., Pseudomonas sp., Burkholderia sp., Alcaligenes sp., Enterobacter sp., Escherichia sp., Klebsiella sp., Proteus sp., Staphylocccus sp., Micrococcus sp., Streptococcus sp., Sarcina sp., Alcaligenes sp., Clostridium sp., and Bacteroides sp.;* or one or more fungi or yeasts selected Rom the genus *Phoma glomerata (Peyronellaea), Aureobasidium sp., Stemphylium sp., Alternaria sp., Aspergillus sp., Botryodiplodia sp., Botrytis sp., Cladosporium sp. Cephalosporium sp., Fusarium sp., Helminthosporium sp., Paeeilomyces sp., Rhizopus sp., Penicillium sp., Candida sp., Geotichum sp.,* and *Saccharomyces sp.* Further examples of living cells comprise one or more bacteria selected from *Bacillus subtilis, Pseudomonas aeruginosa, Burkholderia cepacia,* and *Staphylocccus aureus;* or one or more fungi or yeast selected from *Aureobasidium pullulans, Alternaria alternata, Aspergillus niger, Penicillium chrysogenum* and *Candida albicans.*

Our process releases ATP from living cells in an aqueous dispersion of a polymer or pigment. The term "aqueous dispersion", as used herein, refers to and is intended to be synonymous with simple suspensions or slurries of polymer particles in aqueous liquids, for example, a polyester, polyamide, or polycarbonate particles suspended in an aqueous liquid; aqueous dispersions or emulsions of polymers, such as, for example, acrylic or vinyl latexes, and aqueous solutions of water-soluble polymers, such as aqueous solutions of sulfonated polyesters. The term "aqueous" as used herein refers to any liquid which contains water at a concentration of at least 0.5% by weight based on the total weight of the mixture. For example, in the context of the present invention, an aqueous mixture may comprise water at concentrations of 1 wt%, 5 wt%, 10 wt%, 25 wt%, 50 wt%, 75 wt%, 90 wt%, 95 wt%, or 99 wt%. In addition to water, the aqueous dispersion may contain one or more water-miscible solvents such as, for example, alcohols, ketones, glycols, glycol ethers, esters, and the like. The polymer may comprise one or more polymers which are capable of being suspended, dispersed, emulsified, or dissolved in an aqueous liquid. The term "polymer", as used herein, refers to both homopolymers and copolymers. Examples of polymers include, but are not limited to, vinyl polymers, acrylic polymers, polyesters, polyamides, polycarbonates, polysilanes, polyacrylonitriles, polyolefins, polyethers, polyurethanes, and cellulosics. Additional examples of polymers are one or more sulfonated polyesters, vinyl ester polymers, or acrylic polymers. The aqueous mixture of a polymer or pigment also may comprise an aqueous dispersion, emulsion, latex, or solution of a polymer. For example, latexes can be formed by aqueous emulsion polymerization of one or more ethylenically unsaturated monomers such as styrene, butyl acrylate, methyl methacrylate, vinyl acetate, vinyl 2-ethylhexanoate, acrylic acid, acrylonitrile, glycidyl methacrylate, 2-hydroxyethyl acrylate and the like. Other examples of aqueous mixtures of polymers include aqueous dispersions of vinyl polymers, alkyds, polyesters, acrylics, and uralkyds, and aqueous dispersions and solutions of sulfonated polyesters, polysaccharides, carbohydrates, dispersed cellulosics, and starches.

In aqueous latexes, the polymers generally exist as particles dispersed in water. The particles are generally spherical in shape. The particles may be structured or unstructured. Structured particles include, but are not limited to, core/shell particles and gradient particles. The core/shell polymer particles may also be prepared in a multilobe form, a peanut shell, an acorn form, or a raspberry form. Typically, the core portion contains 20 to 80 wt % of the total weight of the particle and the shell portion contains 80 to 20 wt % of the total weight of the particle. The average particle size of the hybrid latex may range from 25 to 500 nm.

The process of the invention also comprises aqueous dispersions of pigments. Examples of pigments include, but are not limited to, one or more of: titanium dioxide, barium sulfate, copper sulfate, barium chloride, copper chloride, zinc oxide, zinc sulfide, lead carbonate, antimony oxide, or organic pigments familiar to those skilled in the art. Further examples of pigments include the typical organic and inorganic pigments, well-known to one of ordinary skill in the art of surface coatings, especially those set forth by the Colour Index, 3d Ed., 2d Rev., 1982, published by the Society of Dyers and Colourists in association with the American Association of Textile Chemists and Colorists. Examples include, but are not limited to, the following: barytes, clay, CI Pigment White 6 (titanium dioxide); Cl Pigment Red 101 (red iron oxide); CI Pigment Yellow 42; CI Pigment Blue 15, 15:1; 15:2, 15:3, 15:4 (copper phthalocyanines); CI Pigment Red 49:1; and CI Pigment Red 57:1. Figments, in the context of the present invention, may also comprise colorants such as phthalocyanine blue, molybdate orange, TIPURE^{®} R-746 (a titanium pure slurry available from Dupont Chemical, Inc. of Wilmington, Del.).

The aqueous dispersion of a polymer or pigment may comprise one or more components of a coating, an adhesive, a cosmetic, an ink, or a polish. For example, aqueous emulsion polymers or latexes in both clear and pigmented form are well-known. Examples of their uses include interior and exterior architectural coatings, general metal coatings, adhesives, cosmetics and the like. Latex paint compositions, which may comprise the polymers as described above, typically contain one or more leveling, rheology, and flow control agents such as silicones, fluorocarbons or cellulosics; extenders; reactive coalescing aids; plasticizers; flatting agents; pigment wetting and dispersing agents and surfactants; ultraviolet (UV) absorbers; UV light stabilizers; tinting pigments; extenders; defoaming and antifoaming agents; anti-settling, anti-sag and bodying agents; anti-skinning agents; anti-flooding and anti-floating agents; fungicides and mildewcides; corrosion inhibitors; thickening agents; or coalescing agents. Specific examples of such additives can be found in Raw Materials Index, published by the National Paint & Coatings Association, 1500 Rhode Island Avenue, N.W., Washington, D.C. 20005. Further examples of such additives and emulsion polymerization methodology are described in U.S. Patent No. 5,371,148.

Examples of flatting agents include synthetic silica, available from the Davison Chemical Division of W. R. Grace & Company under the trademark SYLOID^{®}; polypropylene, available from Hercules Inc., under the trademark HERCOFLAT^{®}; synthetic silicate, available from J. M. Huber Corporation under the trademark ZEOLEX^{®}.

Examples of dispersing agents and surfactants include sodium bis(tridecyl) sulfosuccinnate, di(2-ethyl hexyl) sodium sulfosuccinnate, sodium dihexylsulfosuccinnate, sodium dicyclohexyl sulfosuccinnate, diamyl sodium sulfosuccinnate, sodium diisobutyl sulfosuccinnate, disodium iso-decyl sulfosuccinnate, disodium ethoxylated alcohol half ester of sulfosuccinnic acid, disodium alkyl amido polyethoxy sulfosuccinnate, tetra-sodium N-(1,2-dicarboxyethyl)-N-octadecyl sulfosuccinnamate, disodium N-octasulfosuccinnamate, sulfated ethoxylated nonylphenol, 2-amino-2-methyl-1-propanol, and the like.

Examples of viscosity, suspension, and flow control agents include polyaminoamide phosphate, high molecular weight carboxylic acid salts of polyamine amides, and alkylene amine salts of an unsaturated fatty acid, all available from BYK Chemie U.S.A. under the trademark ANTI TERRA^{®}. Further examples include polysiloxane copolymers, polyacrylate solution, cellulose esters, hydroxyethyl cellulose, hydrophobically-modified hydroxyethyl cellulose, hydroxypropyl cellulose, polyamide wax, polyolefin wax, carboxymethyl cellulose, ammonium polyacrylate, sodium polyacrylate, and polyethylene oxide. Other examples of thickeners include the methylene/ethylene oxide associative thickeners and water soluble-carboxylated thickeners, for example, those sold under the UCAR POLYPHOBE trademark by Union Carbide.

Several proprietary antifoaming agents are commercially available, for example, under the trademark BRUBREAK of Buckman Laboratories Inc., under the Byk^{®} tradename of BYK Chemie, U.S.A., under the Foamaster^{®} and Nopco^{®} trademarks of Henkel Corp./Coating Chemicals, under the DREWPLUS^{®} trademarks of the Drew Industrial Division of Ashland Chemical Company, under the TRYSOL^{®} and TROYKYD^{®} trademarks of TroyChemical Corporation, and under the SAG^{®} trademarks of Union Carbide Corporation.

Examples of fungicides, mildewcides, and biocides include 4,4-dimethyl-oxazolidine, 3,4,4-trimethyloxazolidine, modified barium metaborate, potassium N-hydroxy-methyl-N-methyldithiocarbamate, 2-(thiocyano-methylthio)benzothiazole, potassium dimethyl dithiocarbamate, adamantane, N-(trichloromethylthio)phthalimide, 2,4,5,6-tetrachloroisophthalonitrile, orthophenyl phenol, 2,4,5-trichlorophenol, dehydroacetic acid, copper naphthenate, copper octoate, organic arsenic, tributyl tin oxide, zinc naphthenate, and copper 8-quinolinate.

Examples ofU.V. absorbers and U.V. light stabilizers include substituted benzophenone, substituted benzotriazoles, hindered amines, and hindered benzoates, available from American Cyanamid Company under the trademark CYASORB UV, diethyl-3-acetyl-4-hydroxy-benzyl-phosphonate, 4-dodecyloxy-2-hydroxy benzophenone, and resorcinol monobenzoate.

The latex compositions may also contain a water-miscible organic solvent and/or coalescing agent. Such solvents and coalescing agents are well known and include ethanol, n-propanol, isopropanol, n-butanol, sec-butanol, isobutanol, ethylene glycol monobutyl ether, propylene glycol n-butyl ether, propylene glycol methyl ether, propylene glycol monopropyl ether, dipropylene glycol methyl ether, diacetone alcohol, TEXANOL^{®} ester alcohol, from Eastman Chemical Company, and the like. Such solvents and coalescing aids may also include reactive solvents and coalescing aids such as, Tor example, diallyl phthalate, and SANTOLINK XI-100^{®} polyglycidyl allyl ether from Monsanto.

The process of the present invention comprises agitating the aqueous mixture in the presence of a particulate disruption agent sufficient to cause rupturing of and thereby release ATP from said living-cells. The term "particulate disruption agent", as used herein, is intended to mean that the disruption agent-comprises any solid particles having a hardness greater than that nf the cell walls of the living cells and such that, when agitated violently in the presence of living cells, the solid particles contact the living cells and thereby rupture their cells. The terms "disrupt", "disrupting", "disruption", "rupture" ,"rupturing", "lyse", or "lysing", are intended to be synonymous and mean that the-cell walls are burst, torn, or ripped open causing the contents of the living-cells to spill into the disruption medium. One of skill in the art will understand that determination of an agitation rate sufficient to cause rupturing can be accomplished by agitating a aqueous sample of a polymer or pigment known to contain living cells in the presence of a particulate disruption agent and detecting the ATP thereby released. In accordance with the present invention, the disruption agent comprises solid particles but may include in addition to the solid particles one or more chemical lysing agents such as, for example, an ionic or nonionic surfactant or detergent. The disruption agent of the invention, however, is not intended to include only a chemical lysing agent in the absence of a particulate disruption agent.

The particulate disruption agent may comprise any material harder than the walls of the living cells. For example the disruption agent may comprise one or more metals, metal oxides, silicon oxide, carborundum, ceramic, glass, plastic, or sand. Although non-dissolvable particles are preferred, a particle with a slow rate of dissolution would also be suitable.

The particulate disruption agent may also be of various shapes, including for example, spheres, cubes, oval, capsule-shaped, tablet-shaped, non-descript random shapes, etc., and may be of uniform shape or non-uniform shapes. It is preferable that the disruption agent is round or oval shaped. Typically, the disruption agent particles have an average diameter of 0.1 to 1 mm. Further examples of average diameters of the disruption agent are 0.1 to 0.7 mm and 0.1 to 0.5 mm. The average diameter of the disruption agent particles may be determined by several methods well known to those skilled in the art such as, for example, examining a small sample of particles by optical microscopy, measuring the diameter of each of particle in the sample (for irregularly shaped particles, at their widest points), and calculating the average particle diameter by dividing the sum of the diameters of all of the particles by the total number of particles in the sample. Although the amount of disruption agent is not critical, typically 0.1 grams to 2 grams of disruption agent may be employed, depending on the sample size of the aqueous mixture of polymer. Preferably, the particulate disruption agent comprises glass beads having an average diameter of 0.1 to 1 millimeter (mm) or, more preferably, 0.1 to 0.5 mm. The glass beads of the present invention may have a broad distribution of diameters or may have a narrow distribution of diameters. It is preferred that the glass bead have a narrow distribution of diameters in which the diameters vary by up to plus or minus 20% of the average diameter. In yet another example, the particulate disruption agent comprises a mixture of one or more sets of glass beads having different average diameters. Examples of average diameters include, but are not limited to 0.1 mm, 0.2 mm, 0.3 mm, 0.4 mm. 0.5 mm, 0.6 mm, 0.7 mm, 0.8 mm, 0.9 mm, and 1.0 mm. In another embodiment, the particulate disruption agent comprises a set of glass beads having an average diameter of 0.1 mm and a set of glass beads having an average diameter of 0.5 mm. Those of skill in the art will appreciate that other sizes, amounts, and combinations can be employed.

The sample of aqueous mixture of polymer and the particulate disruption agent may be placed together in a sample container or vessel of any type or material. Examples of containers include cuvettes, test tubes, and vials of various sizes and shapes (e.g., tubular, rectangular, round, bulb-shaped, etc.). The container may be of any commonly used material such as, for example, glass, plastic, or metal.

The aqueous mixture is agitated in the presence of a particulate disruption agent sufficient to cause rupturing of the cell walls or membranes of the living cells. The agitation may comprise one or more types of movements including, but not limited to, up and down, vibrational, elliptical, or a "figure 8" movement. Preferably, the container of the aqueous mixture and the particulate disruption agent is agitated in a vibrational or oscillating movement. Typically, the aqueous mixture is agitated at a rate of 100 or 10,000 oscillations per minute, 1,000 to 8,000 oscillation per minute, or 2000 to 6000 oscillations per minute. The duration of agitation may be from 0.1 to 10 minutes, from 1 to 7 minutes, or 1 to 3 minutes.

The agitation may be carried out using any number of cell disrupters available commercially. Examples of commercial cell disrupters include the BEAD-BEATER™ and MINI-BEADBEATER™ by Biospec Products (Bartlesville, Okla.) and the WIG-L-BUG™ by Cresent. For example, the agitation may be carried out using a bead mill operated at 100 to 10,000 oscillations per minute for a period of 0.1 to 5 minutes. In another example, the agitation may be conducted using a bead mill operated at 2000 to 6,000 oscillations per minute for a period of 1 to 3 minutes.

One embodiment of our invention is a process for releasing ATP from living cells in an aqueous dispersion of a polymer comprising: agitating said aqueous mixture in the presence of a particulate disruption agent comprising glass beads, metal beads, plastic beads, ceramic beads, metal oxide beads, or sand, at an oscillation rate of 2000 to 6000 oscillations per minute for 1 to 5 minutes thereby releasing said ATP from said living cells, in which said polymer comprises one or more sulfonated polyesters, vinyl ester polymers, or acrylic polymers. Preferably, the disruption agent comprises glass beads having an average diameter of 0.1 to 0.5 mm. In another example, the agitation is carried out using a bead mill operated at 3000 to 5,000 oscillations per minute for a period of 1 to 3 minutes. In another example, the particulate disruption agent comprises a mixture of one or more sets of glass beads having different average diameters. In yet another example, the particulate disruption agent comprises a set of glass beads having an average diameter of 0.1 mm and a set of glass beads having an average diameter of 0.5 mm.

Our process for releasing ATP from living cells may be used in combination with assays for ATP as a method to detect living cells in aqueous dispersions of polymers or pigments. The present invention thus provides a process for detecting living cells in an aqueous dispersion of a polymer or pigment comprising (i) agitating said aqueous mixture in the presence of a particulate disruption agent sufficient to cause rupturing of and release of ATP from the living cells; and (ii) detecting the ATP released in step (i). The polymer may comprise one or more polymers which are capable of being suspended, dispersed, emulsified, or dissolved in an aqueous liquid. Examples of polymers include, but are not limited to, vinyl polymers, polyesters, polyamides, polycarbonates, polysilanes, polyacrylonitriles, polyolefins, polyethers, polyurethanes, or cellulosics. In addition, the polymers may comprise one or more sulfonated polyesters, vinyl ester polymers, or acrylic polymers. The aqueous dispersion of a polymer or pigment also may comprise an aqueous dispersion, emulsion, or solution of a polymer such as, for example, vinyl latex emulsions, acrylic latexes, and water dispersions and solutions of sulfonated polyesters, dispersed cellulosics, starches. The pigment may comprise any pigment as described previously. Examples of pigments include, but are not limited to, one or more of titanium dioxide, barium sulfate, copper sulfate, barium chloride, copper chloride, zinc oxide, zinc sulfide, lead carbonate, antimony oxide, or organic pigments familiar to those skilled in the art.

Preferably, the disruption agent comprises glass beads having an average diameter of 0.1 to 1 mm. More preferably, the disruption agent-comprises glass beads having an average diameter of 0.1 to 0.5 mm. The particulate disruption agent also may comprise a mixture of one or more sets of glass beads having different average diameters; for example, the particulate disruption agent may comprise a set of glass beads having an average diameter of 0.1 mm and a set of glass beads having an average diameter of 0.5 mm.

The agitation, preferably, is carried out using a bead mill operated at 2000 to 6000 oscillations per minute for a period of 1 to 5 minutes. The living cells, typically, will comprise one or more of: prokaryotic cells, eukaryotic cells, plant cells, animal cells, protoplast, spheroplasts, spores, yeasts, fungi, mold, or mycobacteria.

The ATP released from the living cells may be detected by any method known to persons skilled in the art. A preferred method, however, comprises one or more bioluminescent assays such as a luciferin/luciferase ATP assay. The luciferin/luciferase bioluminescent reagent may be prepared using firefly lantern extract or the individual constituents which participate in the bioluminescent reaction. The reaction mixture incorporating individual constituents is a controlled mixture of luciferin, purified luciferase and a Mg⁺². This mixture may be prepared by dissolving luciferin, purified luciferase and MgSO₄ in a sterile aqueous solution. Arsenate and/or other buffers may be added to provide a pH range that will not denature the luciferase enzyme. For example, a reagent may be prepared by dissolving commercial lyophilized firefly lantern extract in a sterile aqueous solution, at a pH of 7.4, having MgSO₄ and a luciferase kinetic modifier such as, for example, potassium arsenate or dithiothreitol, in concentrations of 0.01 M and 0.05 M, respectively. Non-firely luciferases, including those expressed in bacteria that use ATP and luciferin as substrates, also may be used. Alternatively, various other buffers, such as, tris(hydroxymethyl) aminomethane, may be used. Firefly lantern extract may also be obtained in the laboratory from desiccated firefly tails. The firefly tails are first ground to a fine powder with a mortar and pestle with a small amount of washed silica. The powder is then extracted with an arsenate/magnesium sulfate solution at a non-denaturing pH.

Typically, the luciferin/luciferase ATP assay is carried out by mixing 0.05 to 1 gram of a sample of the aqueous mixture of the polymer, after agitation with the particulate disruption agent, with an excess of luciferin (D-luciferin cofactor), luciferase (enzyme) and oxygen in the presence of magnesium ion. The aqueous reaction medium will generally contain enough oxygen to allow the reaction to take place. The reaction of ATP with luciferin produces AMP (adenosine monophosphate), inorganic phosphate, carbon dioxide, and light (photons). The reaction is typically carried out in a transparent container such as, for example, a glass or plastic vial, where the emission of photons can be detected by a luminometer. Because the response (i.e., photon emission) is almost instantaneous, the reaction mixture should be positioned in front of the photon detector as soon as possible after introduction of the bioluminescent reagent. Also, the agitated aqueous mixture and the bioluminescent reagent should be mixed as rapidly as possible. Typically, the emission of photons is detected within 1 to 2 minutes from the end of the disruption step. The bioluminescent response with ATP is determined by integrating the photon response curve or by measuring the maximum intensity of the emitted light, which after reaching this maximum value, decays logarithmically. With all other factors constant, the maximum intensity is directly proportional to the concentration of ATP. Luminometers which may be used for the ATP assay are sold commercially, for example, under the trademark UNILITE XCEL™ luminometer by BioTrace, Inc. The detection of light over a background level is an indication of the presence of living cells and that disruption of the living cells has occurred. This light can be quantified and used to correlate to an amount of ATP. The amount of ATP, however, does not necessarily relate directly to the number of microorganisms or bacterial cells or colonies. ATP testing, however, is useful as a qualitative method to determine the presence of microorganisms.

The luciferase enzyme must be maintained at a pH which does not cause denaturing of the enzyme, typically 6 to 9 pH units, in order to be effective and is usually achieved by employment of a buffer solution. If the proper pH is not maintained, the reaction will not work efficiently, and the results will be erroneous. Luciferase, however, is unstable while in solution, and will degrade, particularly at higher temperatures. Generally, at room temperature, the luciferase solution will remain effective for a period of hours whereas at near freezing temperatures, the luciferase solution will last for a period of days. In addition, luciferin in solution is light sensitive. Light-causes the dissolved luciferin to degrade. Once the luciferin has degraded, no cofactor remains to promote the bioluminescent reaction which may cause false negatives.

Typically, for convenience, the luciferase, luciferin, and magnesium ion are sold as a single combined reagent in a kit. Examples of commercially available kits are CleanTrace^{®}, AquaTrace Free ATP^{®} from BioTrace, Inc., and the Lightning MVP^{®} system by Biocontrol. Many of the commercially available kits contain chemical lysing agents, such as surfactants, in addition to the bioluminescent reagents. As described hereinabove, the presence of chemical lysing agents is not critical to the processes of the present invention but may provide additional lysing or disruption of living cells present in the aqueous mixture of polymers.

Preferably, the disruption agent comprises glass beads having an average diameter of 0.1 to 1 mm. More preferably, the disruption agent comprises glass beads having an average diameter of 0.1 to 0.5 mm. The particulate disruption agent also may comprise a mixture of one or more sets of glass beads having different average diameters, or may comprise a set of glass beads having an average diameter of 0.1 mm and a set of glass beads having an average diameter of 0.5 mm.

The agitation, preferably, is carried out using a bead mill operated at 2000 to 6000 oscillations per minute for a period of 1 to 5 minutes. Thus, a preferred embodiment of our invention is a process for detecting living cells in an aqueous mixture of a polymer comprising: (i) agitating said aqueous mixture with a bead mill operated at 2000 to 6000 oscillations per minute for a period of 1 to 5 minutes in the presence of glass beads having an average diameter of 0.1 to 0.5 mm; (ii) contacting the agitated aqueous mixture from step (i) with a luciferin/luciferase reagent to cause a release of photons; and (iii) measuring the photons released in step (ii); in which said polymer comprises comprises one or more sulfonated polyesters, vinyl ester polymers, or acrylic polymers. The bead mill also may be operated at 3000 to 5000 oscillations per minute for a period of 1 to 3 minutes.

Our process for detecting living cells may be conveniently carried out using a kit having the necessary containers and sampling devices to-disrupt the living cells and to manipulate assay solutions. One aspect of represent invention, therefore, isa process for detecting living cells in an aqueous mixture of a polymer or pigment comprising: (i) agitating said aqueous mixture in a disruption container with a bead mill in the presence of a particulate disruption agent; (ii) withdrawing a sample of said agitated mixture from step (ii) with a sampling device comprising a handle and an adsorbent tip; (iii) inserting said sampling device into an assay container comprising therein a bioluminescent reagent retained by a frangible membrane; (iv) breaking said frangible membrane with said sampling device thereby contacting said sample from step (ii) with a bioluminescent reagent to cause a release of photons; and (v) detecting the photons released in step(iv). Our process may further comprise a kit comprising:
i. a disruption container comprising a disruption agent therein;
ii. a sampling device comprising a handle and an adsorbent tip; and
iii. an assay container comprising therein a bioluminescent reagent retained by a frangible membrane.

The living cells, polymers, cell disrupters, disruption agents, and bioluminescent reagents comprised by these embodiments are as described hereinabove. The disruption container may be any suitable vessel capable of withstanding the violent agitation from the bead mill. The disruption container may be any shape or size, but it is preferable that the container be a glass, metal, or plastic tube or vial. The particulate disruption agent may comprise glass beads having an average diameter of 0.1 to 1 mm. Non-limiting examples of glass beads are those having an average diameter of 0.1 mm, 0.3 mm, and 0.8 mm. More preferably, the disruption agent comprises glass beads having an average diameter of 0.1 to 0.5 mm. The particulate disruption agent also may comprise a mixture of one or more sets of glass beads having different average diameters, or may comprise a set of glass beads having an average diameter of 0.1 mm and a set of glass beads having an average diameter of 0.5 mm. In another embodiment, the particulate disruption agent comprises a set of.glass beads having an average diameter of 0.1 mm and a set of glass beads having an average diameter of 0.5 mm. Those of skill in the art will appreciate that other sizes, amounts, and combinations can be employed.

The agitation, as described previously, may be carried out at any rate-sufficient to cause rupturing of the living cells. Typically, the aqueous mixture is agitated at a rate of 100 or 10,000 oscillations per minute, 1,000 to 8,000 oscillation per minute, or 2000 to 6000 oscillations per minute. The duration of agitation may be from 0.1 to 10 minutes, from 1 to 7 minutes, or 1 to 3 minutes. The ATP released from the living cells may be detected by any method known to persons skilled in the art. A preferred method, however, comprises one or more bioluminescent assays such as, for example, by the luciferin/luciferase ATP assay.

The process of the invention comprises a sampling device, comprising a handle and an absorbent tip, for example, a swab-like device having a stem, handle, and an end thereof, which may be contacted with the agitated aqueous mixture so as to collect a sample. The sampling device may be made of plastic, wood or metal, with the tip made of absorbent material such as cotton, or synthetic material (plastic), or a hollow tube; e.g., a disposable pipette. The tip may be used to obtain a sample by a capillary or vacuum suction, or an affinity probe that can adsorb the analyte by bioaffinity binding; e.g., antibodies or receptors, also may be used.

Our process also comprises an assay container comprising therein a bioluminescent reagent retained by a frangible membrane. The assay container may be any shape or size, but it is preferable that the container be a glass, metal, or plastic tube or vial. Preferably, the assay container is made of a transparent material to permit detection of the released photons by a luminometer directly without transfer of its contents to another container. The assay container generally comprises at least one sealed reagent package containing a bioluminescent reagent, which may be solid, Liquid, powder, emulsion, suspension, tablet or any combination separately or admixtured thereof .

There may be a plurality of separate sealed bioluminescent reagent packages, depending on the particular test method selected for the test sample. The bioluminescent reagent is retained by a frangible membrane such as, for example onion skin glass, carbowax, stiff plastics such as cellophane, certain laminates, plastic coated foils, or any frangible organic compound which is insoluble in the bioluminescent reagent, is compatible with later processing of the sample, and which may punctured, broken, penetrated or unsealed by the longitudinal movement of the sampling device so as to permit the admixture or contacting of the sample and the bioluminescent reagent released from the broken membrane.

After withdrawing a sample of the agitated aqueous dispersion, the sampling device is inserted into an assay container comprising therein a bioluminescent reagent retained by a frangible membrane. The longitudinal or plunging movement of the sampling device breaks said frangible membrane, thereby contacting the agitated aqueous sample with the bioluminescent reagent to cause a release of photons. Typically, after inserting the sampling device into the assay container and releasing the bioluminescent reagent, the assay container is agitated gently for 10 seconds to 2 minutes to completely mix the sample of aqueous mixture and the bioluminescent reagent. The reaction of ATP and the bioluminescent reagent causes a release of photons which may be detected or measured by a luminometer, as described hereinabove, or any other solid state light sensitive detection device known to persons skilled in the art. Typically, the photon emission is detected by the luminometer within 1 to 2 minutes from the end of the disruption step.

Our invention provides yet another process for detecting living cells in an aqueous dispersion of a polymer or pigment comprising: (i) agitating said aqueous dispersion in a disruption container comprising a particulate disruption agent therein; (ii) attaching to said disruption container a reagent container comprising a bioluminescent reagent therein; (iii) contacting said aqueous dispersion with said bioluminescent reagent to cause a release of photons; and (iv) detecting the photons released in step(iii). Our process may further comprise a kit comprising: a disruption container comprising a particulate disruption agent therein; and a reagent container comprising a bioluminescent reagent therein. In yet another embodiment, the process of the invention further comprises a kit comprising: a disruption container comprising a particulate disruption agent therein; and a reagent container comprising therein one or more frangible, meltable, or dissolvable membranes, and a bioluminescent reagent retained by said membranes.

The living cells, polymers, cell disrupters, agitation rate, disruption agent, disruption container, and bioluminescent reagents are as described hereinabove. Preferably the polymers comprise one or more sulfonated polyesters, vinyl ester polymers, or acrylic polymers The disruption container may be any suitable vessel capable of withstanding the violent agitation from the bead mill. The disruption container may be any shape or size, but it is preferable that the container be a glass, metal, or plastic tube or vial. The disruption chamber may have an attachments means such as, for example, threads or a plug in male or female opening which will permit a secure and leak-free connection to the reagent container. The reagent container may also have an attachment means which is compatible with that of the disruption chamber and permits a secure and leak-free transfer of the contents of one container to the other container. The reagent container may be any shape or size, but it is preferable that the container be a glass, metal or plastic tube or vial Preferably, the reagent container is made of a transparent material to permit detection of the released photons by a luminometer directly without transfer of its contents to another container. The reagent container comprises a bioluminescent reagent therein. The bioluminescent reagent may be solid, liquid, powder, emulsion, suspension, tablet or substantially any combination separately or admixtured thereof. Alternatively, the reagent container may comprise therein one or more frangible, meltable, or dissolvable membranes, and a bioluminescent reagent retained by these membranes. In this latter embodiment, the reagent container will typically comprise at least one sealed reagent package containing a bioluminescent reagent.

The bioluminescent reagent may be retained by one or more frangible, meltable, or dissolvable membranes which, for example, may comprise separate sealed bioluminescent reagent packages or compartments containing the bioluminescent reagent within the reagent container separated by transverse membranes. The membranes maybe made of any material designed to retain bioluminescent reagent until its desired release. For example, the membranes may be made of a frangible, dissolvable or meltable material. In the case of a dissolvable membrane material, the material is selected based on the nature of the sample and the desired time of release of the bioluminescent reagent. Gelatin, polyethylene glycol (PEG) and certain sugar materials, for example, may generally be customized by inclusion of particular constituents to dissolve at faster or slower rates. With a meltable membrane material, the material need merely be selected based on its melting profile (temperature and time or rate). For example, when used in a sample processing method that includes a heating step to render infectious organisms noninfectious and/or disrupt cells, a barrier material which melts at temperatures greater than 40°C is suitable as most such heating steps apply temperatures of at least 40°C to the sample. Frangible barrier materials include any materials which will break because of impact of a pellet or particle therewith; impact of the frangible barrier material with an impinging device such as, for example, a wood, metal, or plastic rod; or pressure upon the membrane such as, for example, by squeezing the sides of the reagent container against the frangible barrier. Examples of frangible materials include onion skin glass, carbowax, stiff plastics such as cellophane, certain laminates, plastic coated foils, or any frangible organic compound which will not immediately dissolve in a sample and is compatible with later processing of the sample.

The process of the invention is illustrated further by the following embodiment but persons skilled in the art will understand that other variations are possible. After agitation of aqueous mixture of polymers in the disruption container, the reagent container is attached to the disruption container, by for example, screwing the reagent container onto the disruption container. Alternatively, the two containers could be inserted into one another or simply held together by tape. The bioluminescent reagent within the reagent container is allowed to contact the agitated aqueous sample to cause a release of photons. As an alternative, the bioluminescent reagent within the reagent container is retained by one or more frangible, meltable, or dissolvable membranes and is released by puncturing the membrane with an impinging device. The attached disruption and reagent containers are held in a position to allow the released bioluminescent reagent to flow or fall into the disruption container thereby contacting the agitated aqueous sample with the bioluminescent reagent to cause a release of photons. In another embodiment, the reagent container is made of a flexible plastic and the bioluminescent reagent is retained within a thin glass capsule. The bioluminescent reagent is released by squeezing the reagent container and breaking the capsule. In yet another embodiment, the reagent and disruption containers are not attached and a sample of the aqueous mixture in the disruption container is withdrawn and added to the reagent container which contains a solid bioluminescent reagent retained by a water dissolvable membrane. The membrane dissolves in the aqueous mixture thus releasing the bioluminescent reagent Typically, after releasing the bioluminescent reagent, the assay container is agitated gently for 10 seconds to 2 minutes to complete mix the sample of aqueous mixture and the bioluminescent reagent. The reaction of ATP and the bioluminescent reagent causes a release of photons which may be detected or measured by a luminometer, as described hereinabove, or any other light sensitive detection device known to persons skilled in the art.

The present invention also provides one or more kits for detecting living cells in an dispersion mixture of a polymer. The present invention thus provides a kit comprising: a disruption container comprising a disruption agent therein; a sampling device comprising a handle and an adsorbent tip; and an assay container comprising therein a bioluminescent reagent retained by a frangible membrane. In another embodiment, our invention provides a disruption container comprising a particulate disruption agent therein; and a reagent container comprising a bioluminescent reagent therein. In yet another embodiment our invention provides a process using a kit for detecting living cells in an aqueous dispersion of a polymer or pigment comprising: a disruption container comprising a particulate disruption agent therein; and a reagent container comprising therein one or more frangible, meltable, or dissolvable membranes, and a bioluminescent reagent retained by said membranes. The living cells, polymers, cell disrupters, agitation rate, disruption agent, disruption container, assay container, reagent container, and bioluminescent reagents are as described hereinabove. Preferably, the disruption agent comprises glass beads having an average diameter of 0.1 to I mm. Non-limiting examples of glass beads are those having an average diameter of 0.1 mm, 0.3 mm, and 0.8 mm. The particulate disruption agent also may comprise one or more sets of glass beads having different average diameters. For example, the disruption agent may comprise a set of glass beads having an average diameter of 0.1 mm and a set of glass beads having an average diameter of 0.5 mm. Those of skill in the art will appreciate that other sizes, amounts, and combinations can be employed. The kits may include any bioluminescent assay known to those skilled in the art; however, it is preferred that the bioluminescent assay comprise luciferase.

Our invention is further illustrated by the following examples.

### EXAMPLES

*General procedure for releasing ATP from and detecting living cells in an aqueous mixture of polymers.* A Mini-BeadBeater^{®} bead mill (BioSpec Products, Inc., Bartlesville, OK), a 2 mL microcentrifuge tube with cap, 0.5 mm diameter glass beads, an AquaTrace^{®} Total ATP swab (BioTrace, Inc., Cincinnati, OH), and a UniLite XCEL^{®} luminometer (BioTrace, Inc.) were used. Glass beads and buffer were placed in a 2 mL plastic vial. Alternatively, a vial that is optically transparent in the emission wavelength range of the ATP-driven bioluminescent reaction (500 - 650 nm) may be used. Glass beads with diameters ranging from 0.1 mm to 1 mm were used. Alternatively, beads made of non-giass materials can be used as well as mixtures of different bead sizes and materials. The microcentrifuge tube was filled with latex emulsion alone; approximately 1 mL of water may be added to the centrifuge tube to lower viscosity before the remainder of the microcentrifuge tube volume is filled with latex emulsion. The bead mill was operated at 4800 oscillations per minute for 2 - 5 minutes. After agitation, about a 50µL aliquot was pipetted directly onto an ATP swab surface. Alternatively, the ATP swab can be dipped into the agitated solution. The swab was then pushed into a tube containing a second sealed tube that contains chemical lysing agents, lyopholized luciferase enzyme, and other critical reagents to the bioluminescent reaction. The plunging of the swab pushes the lyophyolized reagents into an attached reaction vial containing a buffered surfactant solution. The swab assembly was shaken for approximately 5 seconds and then placed into the luminometer detection chamber to be read.

### Examples 1-10

Aqueous latex emulsions and raw materials that were suspected of containing microorganisms were examined by bead milling plus ATP bioluminescence, aerobic plate count, and anaerobic plate count. In example 1, the bead milling step was not performed prior to ATP bioluminescence measurement. Bead milling plus ATP bioluminescence was performed as described in the general procedure. Aerobic plate counts and anaerobic plate counts were performed as (per ASTM D2574). Representative data is shown in Table 1. Abbreviations are colony forming units/mL (CFU/mL), relative light units (RLU), plate count (PC), no data collected (NDC), and too numerous to count (TNTC). In less than about 5% of samples, bead milling of the sample prior to ATP bioluminescence measurement decreased RLU output compared to measurement using ATP bioluminescence without bead milling. An example of this result is shown in Table 1, example 5.

**Table 1**

| **Detection of Living Cells in Aqueous Latex Emulsions** | | | | | |
|---|---|---|---|---|---|
| **Example #** | **Sample Type** | **ATP without Bead Milling (RLU)** | **ATP with Bead Milling (RLU)** | **Aerobic PC** | **Anaerobic PC** |
| 1 | Distilled water | 30 | 4487 | 0 | TNTC |
| 2 | Tap water | NDC | 144140 | 0 | TNTC |
| 3 | 2-Ethylhexyl acrylate | NDC | 314 | 0 | TNTC |
| 4 | Acrylic latex | NDC | 602 | 0 | TNTC |
| 5 | Acrylic latex | 659 | 123 | 200 | NDC |
| 6 | Styrene/acrylic latex | 47 | 2592 | NDC | NDC |
| 7 | Acrylic latex | 141 | 2530 | 453 | NDC |
| 8 | Blend of ionic / nonionic sufactants | 6 | 5412 | 304 | NDC |
| 9 | Acrylic latex | 126 | 948 | 0 (after 3 d) | NDC |
| 10 | Texanol® coalescing aid | 140 | 547 | 0 | NDC |

### Examples 11-17

To compare various cell disruption methods, a sample of aqueous latex emulsion of a sulfonated polyester which contained microorganisms was tested by ATP bioluminescence with 4 different methods of cell disruption: a commercially available test kit alone (purchased from Biotrace and which contains chemical lysing agents), ultrasonication, homogenization, and bead milling. The test kit was used for the luciferin/luciferase bioluminescent assay, thus all samples were exposed the lysing agents present in the test kit. For the test kit alone, about 50 µL of emulsion was sampled for ATP measurement with the Biotrace ATP system. For ultrasonic disruption, a 1 mL aliquot of latex emulsion was exposed to ultrasonic pressure waves (Branson Sonifier 450 with 1/8" microtip horn) for about 2 minutes. About 30 µL of ultrasonicated sample was sampled for ATP measurement with the Biotrace ATP system. For homogenizer disruption, a 1 mL aliquot of latex emulsion was treated with a homogenizer (Brinkmann Polytron PT1200 fitted with a PT-DA 1207/2 EC generator) for about 2 minutes. About 50 µL of homogenized sample was sampled for ATP measurement with the Biotrace ATP system. For bead milling disruption, about 1 mL of emulsion was added to a 2 mL tube containing 0.5 mm glass beads. In some cases, 1 mL of water was added to the tube to help reduce the viscosity of the mixture. The tube was capped and placed into a bead mill (Biospec Products Mini BeadBeater-1) and shaken at 4800 RPM for about 2 minutes. After shaking, about 50 µL of emulsion was sampled for ATP measurement with the Biotrace ATP system. Representative data for all methods of disruption and measurement expressed in relative light units (RLU) resulting from the ATP bioluminescence reaction are shown in Table 2.

**Table 2**

| Comparison of Disruption Methods | | |
|---|---|---|
| Example # | Method | Luminometer Response (RLU) |
| 11 | Commercial Test Kit | 5 |
| 12 | Commercial Test Kit | 8 |
| 13 | Ultrasonication | 104 |
| 14 | Homogenization | 24 |
| 15 | Homogenization | 17 |
| 16 | Homogenization | 20 |
| 17 | Bead milling | 9040 |

## Claims

1. A process for detecting living cells in an aqueous dispersion of a polymer or pigment, comprising: (i) agitating said aqueous dispersion in the presence of a particulate disruption agent sufficient to cause rupturing of and thereby release ATP from said living cells and (ii) detecting said released ATP.

2. The process according to claim 1 in which said living cells comprises one or more of: prokaryotic cells, eukaryotic cells, plant cells, animal cells, protoplast, spheroplasts, spores, yeasts, fungi, mold, or mycobacteria.

3. The process according to claim 2 in which said living cells comprise one or more microorganisms from the genus *Bacillus sp., Lactobacillus sp., Citrobacter sp., Serratia sp., Pseudomonas sp., Burkholderia sp., Alcaligenes sp., Enterobacter sp., Escherichia sp., Klebsiella sp., Proteus sp., Staphylacccus sp., Micrococcus sp., Streptococcus sp., Sarcina sp., Alcaligenes sp., Clostridium sp., Sacteroides sp., Phoma glomerata, Aureobasidium sp., Stemphylium sp., Alternaria sp., Aspergillus sp., Botryodiplodia sp., Botrytis sp., Cladosporium sp., Cephalosporium sp., Fusarium sp., Helminthosporium sp., Paeeilomyces sp., Rhizopus sp., Penicillium sp., Candida sp., Geotichum sp.,* or *Saccharomyces sp.*

4. The process according to claim 3 in which said living cells comprise one or more of: *Bacillus subtilis, Pseudomonas aeruginosa, Burkholderia cepacia, Staphylocccus aureus, Aureobasidium pullulans, Alternaria alternata, Aspergillus niger, Penicillium chrysogenum,* or *Candida albicans.*

5. The process according to claim 4 in which said pigment comprises one or more of: titanium dioxide, barium sulfate, copper sulfate, barium chloride, copper chloride, zinc oxide, zinc sulfide, lead carbonate, calcium carbonate, antimony oxide, clay, copper phthalocyanines, red iron oxide, or an organic pigment.

6. The process according to claim 4 in which said polymer comprises one or more of: a vinyl polymer, a polyester, a polyamide, a polycarbonate, a polysilane, a polyacrylonitrile, a polyolefin, a polyether, a polyurethane, or a cellulosic.

7. The process according to claim 6 in which said polymer comprises one or more sulfonated polyesters, vinyl ester polymers, or acrylic polymers.

8. The process according to claim 6 in which said aqueous dispersion comprises an aqueous dispersion of a polyester, an acrylic, an alkyd, or a uralkyd.

9. The process according to claim 8 in which said agitation is carried out using a bead mill operated at 3000 to 5,000 oscillations per minute for a period of 1 to 3 minutes; said aqueous mixture comprises one or more components of a coating, an adhesive, a cosmetic, an ink, or a polish; and said disruption agent is round or oval shaped and comprises one or more metals, metal oxides, silicon oxide, carborundum, ceramic, glass, plastic, or sand.

10. The process according to claim 9 in which said disruption agent comprises glass beads having an average diameter of 0.1 to .5 mm.

11. The process according to claim 9 in which said disruption agent comprises a mixture of one or more sets of glass beads having different average diameters.

12. The process according to 11 in which said particulate disruption agent comprises a set of glass beads having an average diameter of 0.1 mm and a set of glass beads having an average diameter of 0.5 mm.

13. The process according to claim 1 in which said living cells comprise one or more of: prokaryotic cells, eukaryotic cells, plant cells, protoplasts, spheroplasts, spores, yeasts, fungi, mold, or mycobacteria; said aqueous mixture comprises an aqueous dispersion or solution of one or more sulfonated polyesters, vinyl ester polymers, or acrylic polymers; said disruption agent comprises glass beads having an average diameter of 0.1 to 1 mm; and said agitation is carried out using a bead mill operated at 2000 to 6000 oscillations per minute for a period of 1 to 5 minutes.

14. The process according to claim 13 in which said particulate disruption agent comprises a mixture of one or more sets of glass beads having different average diameters and said detection of said ATP comprises a luciferin/luciferase ATP assay.

15. The process according to claim 13 further comprising: (i) agitating said aqueous dispersion in a disruption container comprising said particulate disruption agent therein; (ii) attaching to said disruption container a reagent container comprising a bioluminescent reagent therein; (iii) contacting said aqueous mixture with said bioluminescent reagent to cause a release of photons; and (iv) detecting the photons released in step(iii).

16. The process according to claim 15 further comprising a kit comprising: a disruption container comprising a particulate disruption agent therein; and a reagent container comprising a bioluminescent reagent therein.

17. A kit for detecting living cells in an aqueous dispersion of a polymer or pigment, comprising a disruption container comprising
(i) a disruption container comprising a particulate disruption agent therein;
(ii) a sampling device comprising a handle and an adsorbent tip; and
(iii) an assay container comprising therein a bioluminescent reagent retained by a frangible membrane.

18. The kit according to claim 17 in which said particulate disruption agent comprises a mixture of one or more sets of glass beads having different average diameters and said bioluminescent reagent comprises luciferase.

## Patentansprüche

1. Verfahren zum Nachweis von lebenden Zellen in einer wässrigen Dispersion eines Polymers oder Pigments, umfassend: (i) Umwälzen der wässrigen Dispersion in Anwesenheit eines teilchenförmigen Aufbrechungsmittels, das ausreichend ist, um ein Aufbrechen der lebenden Zellen und **dadurch** eine Freisetzung von ATP aus denselben zu bewirken, und (ii) Nachweisen des freigesetzten ATP.

2. Verfahren nach Anspruch 1, in dem die lebenden Zellen eine oder mehrere der folgenden umfassen: prokaryotische Zellen, eukaryotische Zellen, Pflanzenzellen, Protoplast, Sphäroplasten, Sporen, Hefen, Pilze, Schimmelpilz oder Mykobakterien.

3. Verfahren nach Anspruch 2, in dem die lebenden Zellen einen oder mehrere Mikroorganismen aus der Gattung *Bacillus sp., Lactobacillus sp., Citrobacter sp., Serratia sp., Pseudomonas sp., Burkholderia sp., Alcaligenes sp., Enterobacter sp., Escherichia sp., Klebsiella sp., Proteus sp., Staphylococcus sp., Micrococcus sp., Streptococcus sp., Sarcina sp., Alcaligenes sp., Clostridium sp., Bacteroides sp., Phoma glomerata, Aureobasidium sp., Stemphylium sp., Alternaria sp., Aspergillus sp., Botryodiplodia sp., Botrytis sp., Cladosporium sp., Cephalosporium sp., Fusarium sp., Helminthosporium sp., Paeeilomyces sp., Rhizopus sp., Penicillium sp., Candida sp., Geotichum sp.* oder *Saccharomyces sp.* umfassen.

4. Verfahren nach Anspruch 3, in dem die lebenden Zellen eine oder mehrere der folgenden umfassen: *Bacillus subtilis, Pseudomonas aeruginosa, Burkholderia cepacia, Staphylococcus aureus, Aureobasidium pullulans, Alternaria alternata, Aspergillus niger, Penicillum chrysogenum* oder *Candida albicans*

5. Verfahren nach Anspruch 4, in dem das Pigment eines oder mehrere der folgenden umfasst: Titandioxid, Bariumsulfat, Kupfersulfat, Bariumchlorid, Kupferchlorid, Zinkoxid, Zinksulfid, Bleicarbonat, Calciumcarbonat, Antimonoxid, Ton, Kupferphthalocyanine, rotes Eisenoxid oder ein organisches Pigment.

6. Verfahren nach Anspruch 4, in dem das Polymer eines oder mehrere der folgenden umfasst: ein Vinylpolymer, einen Polyester, ein Polyamid, ein Polycarbonat, ein Polysilan, ein Polyacrylnitril, ein Polyolefin, einen Polyether, ein Polyurethan oder ein Cellulose-Derivat.

7. Verfahren nach Anspruch 6, in dem das Polymer ein(en) oder mehrere sulfonierte Polyester, Vinylester-Polymere oder Acryl-Polymere umfasst.

8. Verfahren nach Anspruch 6, in dem die wässrige Dispersion eine wässrige Dispersion eines Polyesters, eines Acryl-Harzes, eines Alkyds oder eines Uralkyds umfasst.

9. Verfahren nach Anspruch 8, in dem das Umwälzen über eine Zeitspanne von 1 bis 3 Minute unter Verwendung einer Kugelmühle durchgeführt wird, die bei 3000 bis 5000 Oszillationen pro Minute betrieben wird; die wässrige Mischung eine oder mehrere Komponenten von einem Beschichtungsmittel, einem Klebstoff, einem Kosmetikum, einer Tinte/Druckfarbe oder einem Poliermittel umfasst; und das Aufbrechungsmittel von runder oder ovaler Form ist und ein oder mehrere Metalle, Metalloxide, Siliciumoxid, Carborundum, Keramik, Glas, Kunststoff oder Sand umfasst.

10. Verfahren nach Anspruch 9, in dem das Aufbrechungsmittel Glasperlen mit einem durchschnittlichen Durchmesser von 0,1 bis 0,5 mm umfasst.

11. Verfahren nach Anspruch 9, in dem das Aufbrechungsmittel eine Mischung von einem oder mehreren Sätzen von Glasperlen mit unterschiedlichen durchschnittlichen Durchmessern umfasst.

12. Verfahren nach Anspruch 11, in dem das teilchenförmige Aufbrechungsmittel einen Satz von Glasperlen mit einem durchschnittlichen Durchmesser von 0,1 mm und einen Satz von Glasperlen mit einem durchschnittlichen Durchmesser von 0,5 mm umfasst.

13. Verfahren nach Anspruch 1, in dem die lebende Zelle eine oder mehrere der folgenden umfasst: prokaryotische Zellen, eukaryotische Zellen, Pflanzenzellen, Protoplasten, Sphäroplasten, Sporen, Hefen, Pilze, Schimmelpilze oder Mykobakterien; die wässrige Mischung eine wässrige Dispersion oder Lösung von einem oder mehreren sulfonierten Polyestern, Vinylester-Polymeren oder Acryl-Polymeren umfasst; das Aufbrechungsmittel Glasperlen mit einem durchschnittlichen Durchmesser von 0,1 bis 1 mm umfasst, und das Umwälzen unter Verwendung einer Kugelmühle, die bei 2000 bis 6000 Oszillationen pro Minute betrieben wird, über eine Zeitspanne von 1 bis 5 Minuten durchgeführt wird.

14. Verfahren nach Anspruch 13, in dem das teilchenförmige Aufbrechungsmittel eine Mischung von einem oder mehreren Sätzen von Glasperlen mit unterschiedlichen durchschnittlichen Durchmessern umfasst und der Nachweis des ATP einen Luciferin/Luciferase-ATP-Assay umfasst.

15. Verfahren nach Anspruch 13, weiter umfassend: (i) Umwälzen der wässrigen Dispersion in einem Aufbrechungsbehälter, der darin das teilchenförmige Aufbrechungsmittel umfasst; (ii) Anbringen eines Reagensbehälters, der ein Biolumineszenz-Reagens darin umfasst, an dem Aufbrechungsbehälter; (iii) In-Kontakt-Bringen der wässrigen Mischung mit dem Biolumineszenz-Reagens, um die Freisetzung von Photonen zu bewirken; und (iv) Nachweisen der im Schritt (iii) freigesetzten Photonen.

16. Verfahren nach Anspruch 15, weiter umfassend ein Kit, welches umfasst:
einen Aufbrechungsbehälter, der darin ein teilchenförmiges Aufbrechungsmittel umfasst; und einen Reagensbehälter, der darin ein Biolumineszenz-Reagens umfasst.

17. Kit zum Nachweisen von lebenden Zellen in einer wässrigen Dispersion eines Polymers oder Pigments, umfassend einen Aufbrechungsbehälter, welcher umfasst
(i) einen Aufbrechungsbehälter, der darin ein teilchenförmiges Aufbrechungsmittel umfasst;
(ii) eine Probeentnahmevorrichtung, die einen Handgriff und eine adsorbierende Spitze umfasst; und
(iii) einen Assaybehälter, der darin ein Biolumineszenz-Reagens umfasst, das durch eine zerbrechliche Membran zurückgehalten wird.

18. Kit nach Anspruch 17, in dem das teilchenförmige Aufbrechungsmittel eine Mischung von einem oder mehreren Sätzen von Glasperlen mit unterschiedlichen durchschnittlichen Durchmessern umfasst und das Biolumineszenz-Reagens Luciferase umfasst.

## Revendications

1. Procédé pour la détection de cellules vivantes dans une dispersion aqueuse d'un polymère ou d'un pigment, comprenant : (i) l'agitation de ladite dispersion aqueuse en présence d'un agent de lyse à particules suffisant pour provoquer leur lyse et donc libérer l'ATP desdites cellules vivantes et (ii) la détection dudit ATP libéré.

2. Procédé selon la revendication 1, dans lequel lesdites cellules vivantes comprennent un ou plusieurs parmi : des cellules procaryotes, des cellules eucaryotes, des cellules végétales, des cellules animales, des protoplastes, des sphéroplastes, des spores, des levures, des champignons, des moisissures ou des mycobactéries.

3. Procédé selon la revendication 2, dans lequel lesdites cellules vivantes comprennent un ou plusieurs microorganismes du genre *Bacillus sp., Lactobacillus sp., Citrobacter sp., Serratia sp., Pseudomonas sp., Burkholderia sp., Alcaligenes sp., Enterobacter sp., Escherichia sp., Klebsiella sp., Proteus sp., Staphylocccus sp., Micrococcus sp., Streptococcus sp., Sarcina sp., Alcaligenes sp., Clostridium sp., Bacteroides sp.; Phoma glomerata, Aureobasidium sp., Stemphylium sp., Alternaria sp., Aspergillus sp., Botryodiplodia sp., Botrytis sp., Cladosporium sp., Cephalosporium sp., Fusarium sp., Helminthosporium sp., Paeeilomyces sp., Rhizopus sp., Penicillium sp., Candida sp., Geotichum sp.,* ou *Saccharomyces sp.*

4. Procédé selon la revendication 3, dans lequel lesdites cellules vivantes comprennent un ou plusieurs parmi *: Bacillus subtilis, Pseudomonas aeruginosa, Burkholderia cepacia, Staphylocccus aureus, Aureobasidium pullulans, Alternaria alternata, Aspergillus niger, Penicillium chrysogenum,* ou *Candida albicans.*

5. Procédé selon la revendication 4, dans lequel ledit pigment comprend un ou plusieurs parmi : le dioxyde de titane, le sulfate de baryum, le sulfate de cuivre, le chlorure de baryum, le chlorure de cuivre, l'oxyde de zinc, le sulfure de zinc, le carbonate de plomb, le carbonate de calcium, l'oxyde d'antimoine, l'argile, les phtalocyanines de cuivre, l'oxyde de fer rouge ou un pigment organique.

6. Procédé selon la revendication 4, dans lequel ledit polymère comprend un ou plusieurs parmi : un polymère vinylique, un polyester, un polyamide, un polycarbonate, un polysilane, un polyacrylonitrile, une polyoléfine, un polyéther, un polyuréthane, ou un produit cellulosique.

7. Procédé selon la revendication 6, dans lequel ledit polymère comprend un ou plusieurs polyesters sulfonés, polymères d'esters vinyliques ou polymères acryliques.

8. Procédé selon la revendication 6, dans lequel ladite dispersion aqueuse comprend une dispersion aqueuse d'un polyester, d'un acrylique, d'un alkyde, ou d'un uralkyde.

9. Procédé selon la revendication 8, dans lequel ladite agitation est réalisée en utilisant un broyeur à billes fonctionnant de 3000 à 5000 oscillations par minute pendant une durée de 1 à 3 minutes ; ledit mélange aqueux comprend un ou plusieurs composants parmi un enrobant, un adhésif, un cosmétique, une encre ou un produit à polir ; et ledit agent de lyse est de forme ronde ou ovale et comprend un ou plusieurs métaux, oxydes de métaux, oxyde de silicium, carborundum, céramique, verre, matière plastique ou sable.

10. Procédé selon la revendication 9, dans lequel ledit agent de lyse comprend des billes de verre d'un diamètre moyen de 0,1 à 0,5 mm.

11. Procédé selon la revendication 9, dans lequel ledit agent de lyse comprend un mélange d'une ou plusieurs séries de billes de verre ayant différents diamètres moyens.

12. Procédé selon la revendication 11, dans lequel ledit agent de lyse à particules comprend une série de billes de verre ayant un diamètre moyen de 0,1 mm et une série de billes de verre ayant un diamètre moyen de 0,5 mm.

13. Procédé selon la revendication 1, dans lequel lesdites cellules vivantes comprennent un ou plusieurs parmi : des cellules procaryotes, des cellules eucaryotes, des cellules végétales, des protoplastes, des sphéroplastes, des spores, des levures, des champignons, des moisissures ou des mycobactéries ; ledit mélange aqueux comprend une dispersion ou une solution aqueuse d'un ou plusieurs polyesters sulfonés, polymères d'esters vinyliques ou polymères acryliques ; ledit agent de lyse comprend des billes de verre d'un diamètre moyen de 0,1 à 1 mm ; et ladite agitation est réalisée en utilisant un broyeur à billes fonctionnant de 2000 à 6000 oscillations par minute pendant une durée de 1 à 5 minutes.

14. Procédé selon la revendication 13, dans lequel ledit agent de lyse à particules comprend un mélange d'une ou plusieurs séries de billes de verre ayant différents diamètres moyens et ladite détection dudit ATP comprend une analyse d'ATP luciférine/luciférase.

15. Procédé selon la revendication 13, comprenant en outre : (i) l'agitation de ladite dispersion aqueuse dans un conteneur pour lyse contenant ledit agent de lyse à particules ; (ii) la fixation audit conteneur pour lyse d'un conteneur de réactif contenant un réactif bioluminescent ; (iii) la mise en contact dudit mélange aqueux avec ledit réactif bioluminescent pour provoquer une libération de photons ; et (iv) la détection des photons libérés dans l'étape (iii).

16. Procédé selon la revendication 15, comprenant en outre une trousse comprenant : un conteneur pour lyse contenant un agent de lyse à particules ; et un conteneur de réactif contenant un réactif bioluminescent.

17. Trousse pour la détection de cellules vivantes dans une dispersion aqueuse d'un polymère ou d'un pigment, comprenant un conteneur pour lyse comprenant
(i) un conteneur pour lyse contenant un agent de lyse à particules ;
(ii) un dispositif d'échantillonnage comprenant une poignée et une extrémité adsorbante ; et
(iii) un conteneur pour analyse contenant un réactif bioluminescent retenu par une membrane pouvant se rompre.

18. Trousse selon la revendication 17, dans laquelle ledit agent de lyse à particules comprend un mélange d'une ou plusieurs séries de billes de verre ayant différents diamètres moyens et ledit réactif bioluminescent comprend de la luciférase.
